# EUROPEAN PATENT SPECIFICATION

(11) **EP 0 471 767 B1**
(45) Date of publication and mention of the grant of the patent: **11.09.1996**
(21) Application number: 90908058.2
(22) Date of filing: 24.04.1990
(51) Int. Cl.: B32B 7/12

(54) **TEMPERATURE-ACTIVATED ADHESIVE ASSEMBLIES**
VON DER TEMPERATUR AKTIVIERTE BINDEMITTELEINHEITEN
ENSEMBLES ADHESIFS ACTIVES PAR LA TEMPERATURE

(30) Priority: 11.05.1989 US 350723; 23.03.1990 US 497940
(43) Date of publication of application: 26.02.1992
(73) Proprietor: LANDEC CORPORATION, Menlo Park California 94025 (US)
(72) Inventor: STEWART, Ray, F., Redwood City, CA 94061 (US); SCHMITT, Edward, Emil, Palo Alto, CA 94306 (US)
(74) Representative: Hall, Robert Leonard
(86) International application number: PCT/US90/02223
(87) International publication number: WO 90/13420

(56) References cited:
- EP-A- 0 083 499
- WO-A-91/14461
- US-A- 3 635 754
- US-A- 3 763 858
- US-A- 4 199 646
- US-A- 4 323 557
- US-A- 4 675 009
- US-A- 4 880 683
- US-A- 4 925 908

## Description

This invention relates to adhesive compositions and assemblies.

Pressure-sensitive adhesives (PSA's) are well known -- see for example Encyclopedia of Polymer Science and Engineering, 13, (New York: John Wiley & Sons, 1988), 345-368, and Handbook of Pressure-Sensitive Adhesive Technology, ed. Donates Satas (New York: Van Nostrand Reinhold Co., Inc., 1982). However, they suffer from two problems. First, because they are usually tacky at room temperature, they tend to stick in unwanted positions. Second, they are difficult to remove cleanly from a substrate, particularly if they have been in place for some time. These problems are particularly acute in the medical field.

US-A-3,635,754 describes an adhesive product which is not a PSA at room temperature, but can be converted into a PSA by heating and then remains a PSA for several hours even if maintained at room temperature. The adhesive contains a polyolefin having a melting point of at least 35°C and is activated by heating to a temperature about 10°C above the melting point. The polyolefins in question are prepared by polymerizing an olefin, e.g. dodecene, using a Ziegler-Natta catalyst. The range over which such polymers melt is not discussed in US-A-3,635,754, but is in fact about 20°C or more.

We have now discovered that the problems noted above can be solved through the use of a crystalline polymer which melts over a range of at most 15°C, preferably at most 10°C, particularly at most 5°C, and which begins to melt at a temperature Tₒ of 5 to 50°C, e.g. 20 to 35°C, preferably at least 25°C, particularly 25 to 30°C. Such polymers can be used to provide compositions which are substantially non-tacky at temperatures below Tₒ, preferably at room temperature, and become tacky when heated from a temperature below Tₒ to a temperature at which melting is complete.

Thus, in a first aspect, this invention provides a temperature-sensitive adhesive assembly which comprises
(1) a body member; and
(2) secured to the body member, a layer of a polymeric composition which
   (a) comprises 40 to 100% by weight of at least one crystalline polymer which (i) begins to melt at a temperature Tₒ which is 50 to 50°C, and (ii) completes melting at a temperature Tₘ which is at most (Tₒ+15)°C,
   (b) is substantially non-tacky at temperatures below Tₒ, and
   (c) becomes tacky when heated from a temperature below Tₒ to a temperature above Tₘ.

Such assemblies are particularly useful in medical applications when Tₒ is above room temperature and Tₘ is less than skin temperature, since they will not adhere to the body while they are being positioned, but will adhere when heated by being pressed against the skin. Preferably, the composition develops tack within less than 1 minute when heated above Tₘ and, when cooled to a few degrees below Tₒ without contacting any substrate, reverts to the non-tacky state in less than about 5 minutes.

In a second aspect, this invention provides a method of securing a body member to a substrate which comprises pressing against the substrate the layer (2) of an adhesive assembly according to the first aspect of the invention, while said layer is tacky. Preferably, the layer (2) is heated to a temperature less than 45°C, for example through use of a hair dryer. When the substrate is human skin, the skin itself may heat the layer (2) sufficiently, e.g. to a temperature of about 37°C, and heating to a temperature above 45°C may not be tolerable to the skin. In this method, the assembly, while still at a temperature of less than Tₒ, preferably at room temperature, can be prepositioned (i.e. maneuvered freely on and adjacent to the substrate), and then heated and pressed against the substrate to secure it to the substrate at a desired position.

Preferably the crystalline polymer will have a crystallization point T_{c} of less than skin temperature, preferably 10 to 28°C, particularly 15 to 25°C. When such a polymer is used, the composition will tend to lose tack when cooled, e.g. by ice or a cold pack. Thus, if the assembly has been applied to human skin, or other suitable substrate, and is then cooled, it can thereafter be easily removed. Rapid crystallization can be promoted by the presence of seeding agents or crystallization catalysts.

In a third aspect, this invention provides a method of removing a body member from a substrate, the body member being part of a temperature-sensitive assembly according to the first aspect of the invention which is adhered to the substrate through the layer (2), which method comprises (A) cooling the assembly to a temperature at which the layer (2) is substantially non-tacky, and (B) removing the body member from the substrate.

The values of Tₒ, Tₘ and T_{c} and heats of fusion (ΔH_{f}) referred to herein are determined by means of a differential scanning calorimeter (DSC) at a heating rate of 10°C/min for Tₒ and Tₘ, and at a cooling rate of 10°C/min for T_{c}.

As previously noted, the invention is particularly useful for adhering articles ("body members") to human skin. The article may be flexible or non-flexible, e.g. a woven fabric, non-woven fabric, paper, synthetic film, surgical dressing, adhesive bandage with a gauze pad (which may be medicated) in the center, transdermal drug delivery patch, EKG (or other) electrode, or an immobilization device (e.g. a cast or splint). The body member may be a label, price tag or tape, which may have the great advantage that it can be easily and completely removed from a substrate of metal, glass or plastic merely by chilling. When the novel assemblies are applied to a porous substrate, e.g. paper, cloth or wood, the heated adhesive composition will penetrate the substrate and the bond will remain strong even after cooling.

When the composition is slightly tacky at room temperature, the assembly may include a release liner which protects the layer (2) prior to use. The composition may include a standard PSA or a tackifier to provide it with slight tack at room temperature.

The tack of an adhesive film coating is affected by its thickness, especially when the coating is less than about 0.13 mm (0.005 inch) thick. Generally, tack increases linearly with thickness. In order to compare adhesives having different coating thicknesses, tack values can be (and have been in this application) "normalized", i.e. adjusted to the values which would be expected if the coating of the adhesive was 0.025 mm (0.001 inch) thick. The terms (1) "substantially nontacky", (2) "slightly tacky" and (3) "tacky" are used herein to denote normalized tack values (measured by the PKI method described in detail below) of (1) the minimum limit of the instrument or less than 25 g.cm/sec; (2) a value between that described in (1) and 100 g.cm/sec; and (3) at least 100 g.cm/sec, respectively.

The term "peel strength" is used herein to denote the bond strength between (i) an adhesive-coated article and (ii) a substrate to which the article is joined, when the article and the substrate are separated at a rate of 305 mm/min (12 inches/min) and at an angle between the separated components of about 180°. The peel strength of an adhesive film coating is also affected by its thickness, especially when the coating is less than 0.13 mm (0.005 inch) thick. Generally, peel strength of any particular adhesive increases linearly with the square root of thickness. In order to compare adhesives with different thicknesses, peel strength values can be (and have been in this application) "normalized", i.e. adjusted to the values which would be expected if the coating of the adhesive was 0.025 mm (0.001 inch). A detailed description of two methods of measuring peel strength is given below.

The polymers used in this invention preferably have a weight average molecular weight (M_{w}) of 20,000 to 2,300,000 Daltons, e.g. 100,000 to 1,300,000 Daltons, most typically 250,000 to 1,000,00 Daltons. A blend of two or more different polymers can be used.

The polymers used in this invention can be side chain crystallizable polymers (usually referred to herein as SCC polymers) or main chain crystallizable polymers, with SCC polymers usually being preferred. SCC polymers are well known and are available commercially. SCC polymers are reviewed in J. Polymer Sci.: Macromol. Rev. 8:117-253 (1974), and J. Polymer Sci., Polymer Chemistry Edition, 19, 1871-1873 (1981). In general, SCC polymers contain 50 to 100% by weight of units of the formula 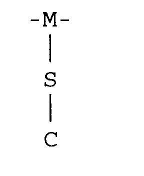 wherein M is a backbone atom, S is a spacer unit and C is a crystallizable group. These polymers generally have a heat of fusion (ΔH_{f}) of at least 20 J/g, preferably at least 40 J/g. If the polymer contains less than 100% X, it will in addition contain units which may be represented by "Y" or "Z", or both, wherein Y is derived from any polar or nonpolar monomer or mixture of polar or nonpolar monomers capable of polymerizing with X and/or Z, and wherein Z is a polar monomer or other units are derived from polar monomers, e.g., polyoxyalkylenes, acrylates, including hydroxyethylacrylate, acrylamides and methacrylamides, their presence will typically increase adhesion to most substrates. If the polymer contains units derived from acrylic acid, it is preferred that such units constitute 1 to 10 wt.% of the polymer.

The backbone of the polymer (defined by "M")may be any organic structure (aliphatic or aromatic hydrocarbon, ester, ether, amide, etc.) or an inorganic structure (sulfide, phosphazine, silicone, etc.), and may include spacer linkages which can be any suitable organic or inorganic unit, for example ester, amide, hydrocarbon, phenyl, ether, or ionic salt (e.g., a carboxyl-alkyl ammonium or sulfonium or phosphonium ion pair or other known ionic salt pair).

The side-chain (defined by "S" and "C") may be aliphatic or aromatic or a combination of aliphatic and aromatic, but must be capable of entering into a crystalline state. Common examples are: linear aliphatic side-chains of at least 10 carbon atoms, derived for example from C₁₄ to C₂₂ acrylates, methacrylates, acrylamides, methacrylamides, vinyl ethers, vinyl esters, siloxanes or alpha olefins; fluorinated aliphatic side-chains of at least 6 carbons; and p-alkyl styrene side-chains wherein the alkyl is of 8 to 24 carbon atoms.

The length of the side-chain moiety is usually greater than 5 times the distance between side-chains in the case of acrylates, methacrylates, vinyl esters, acrylamides, methacrylamides, vinyl ethers and alpha olefins. In the extreme case of a fluoroacrylate alternate copolymer with butadiene, the side-chain can be as little as two times the length as the distance between the branches. In any case, the side-chain units should make up greater than 50% of the volume of the polymer, preferably greater than 65% of the volume.

Specific examples of side-chain crystallizable monomers are the acrylate, fluoroacrylate, methacrylate and vinyl ester polymers described in J. Poly. Sci. 10:3347 (1972); J. Poly. Sci. 10:1657 (1972); J. Poly. Sci. 9:1835 (1971); J.A.C.S. 76:6280 (1954); J. Poly. Sci. 7:3053 (1969); Polymer J. 17:991 (1985), corresponding acrylamide, substituted acrylamide and maleimide polymers (J. Poly. Sci.: Poly. Physics Ed.J. Poly. Sci.: Poly. Physics Ed. 18:2197 (1980); poly(α-olefin) polymers such as those described in J. Poly. Sci.: Macromol. Rev. 8:117-253 (1974) and Macromolecules 13:12 (1980), polyalkylvinylethers, polyalkylethylene oxides such as those described in Macromolecules 13:15 (1980), alkylphosphazene polymers, polyamino acids such as those described in Poly. Sci. USSR 21:241, Macromolecules 18:2141, polyisocyanates such as those described in Macromolecules 12:94 (1979), polyurethanes made by reacting amine- or alcohol-containing monomers with long-chain alkyl isocyanates, polyesters and polyethers, polysiloxanes and polysilanes such as those described in Macromolecules 19:611 (1986), and p-alkylstyrene polymers such as those described in J.A.C.S. 75:3326 (1953) and J. Poly. Sci. 60:19 (1962).

Of specific utility are polymers which are both relatively polar and capable of crystallization, but wherein the crystallizing portion is not affected by moisture. For example, incorporation of polyoxyethylene, polyoxypropylene, polyoxybutylene or copolyoxyalkylene units in the polymer will make the polymer more polar, improving adhesion to moist skin.

In a particularly preferred embodiment, -C is -(CH₂)ₙ-CH₃ or -(CF₂)ₙ-CF₂H, where n is 8 to 20 inclusive, -S- is -O-, -CH₂-, -(CO)-, -O(CO)- or -NR- where R is hydrogen or lower alkyl (1-6 carbons), and -M- is -[(CH₂)ₘ-CH]- where m is 0 to 2.

Typical "Y" units include units derived from linear or branched alkyl or aryl acrylates or methacrylates, alpha olefins, linear or branched alkyl vinyl ether or vinyl esters, maleic esters or itaconic acid esters, acrylamides, styrenes or substituted styrenes, acrylic acid, methacrylic acid and hydrophilic monomers as detailed in WO84/0387.

The polymer may also contain units of the formula 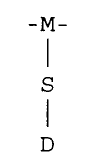 wherein "D" is a hydrophilic polyether chain such as a polyoxyalkylene chain (e.g., polyoxyethylene) which may or may not be crystallizable. "D" preferably has a molecular weight higher than about 100 Daltons.

Polyolefins can exist in a plurality of tactic forms, and in order to obtain the desired sharpness of the transition between tacky and nontacky states, the tacticity of the polymer must be carefully selected. The polymer can be present in a singular configuration, i.e. either a tactic, syndiotactic or isotactic, but not in a mixture of tacticities unless their melting points opportunistically coincide. Having a mixture of various tactic polymers with different melting points will broaden the transition and cause the resultant polymer to exhibit sluggish adhesive property changes over a narrow temperature range.

Preferred main-chain crystallizable polymers include water-insoluble polyalkylene oxides, lower alkyl polyesters and polytetrahydrofuran.

The crystallizable polymer, whether side-chain or main-chain crystallizable, may or may not be crosslinked. Crosslinking the adhesive composition and employing high molecular weight polymers will, in general, result in a material that exhibits decreased melt flow and greater cohesive strength than non-crosslinked and low molecular weight materials. Because the adhesive composition may be used at temperatures above the melting point of the polymer, low melt flow is desirable so that the adhesive will not migrate, flow or transfer to the substrate surface (i.e., in contrast to conventional "hot-melt" adhesives). Adhesive compositions with sufficient cohesive strength to prevent cohesive failure are desirable. Low melt flow and suitable cohesive strength may be achieved by other means such as the addition of suitable co-monomers (e.g., high Tg monomers), block copolymerization, or crosslinking before, during or after preparation of the adhesive assembly.

A variety of methods are available to produce crosslinked crystallizable materials. A network copolymer can be prepared by polymerizing a crystallizable monomer and a multifunctional monomer either in one or two steps. A one-step process may be used to form an adhesive in place, while a two-step process is useful where an intermediate processing step is necessary. A variety of multifunctional monomers (di, tri- or multifunctional acrylic or methacrylic esters, vinyl ethers, esters or amides, isocyanates, aldehydes, epoxies and the like) are known in the art. These multifunctional monomers can be used in a one-or two-step process depending on the desired result. Ionizing radiation, for example beta or gamma radiation, peroxides, silanes, or similar cure agents, can be used to crosslink a preformed crystallizable polymer with or without added comonomers. Ionic crosslinks can be formed by, for example, reacting an acidic polymer site with a di- or trivalent metal salt or oxide to produce a complex which serves as a crosslink site. Likewise, organic salts or complexes can be prepared by methods known in the art.

If the material is crosslinked to too great an extent, crystallinity and/or tack may be decreased to the point that the desirable temperature-activated properties are lost. To optimize the aforementioned factors, crosslinking should be in the range of about 0.01 percent to 5 mole percent, preferably .05 to 1 mole percent. The crosslinked polymers will normally have a heat of fusion of at least 20 J/g, preferably at least 30 J/g.

The effect of crosslinking may also be obtained by using a block copolymer containing a crystallizable portion and a second portion which exhibits a glass transition or melting point higher than the crystallizable portion, the resulting product exhibiting mechanical stability above the melting point of the crystallizable polymer but below the transition of the second polymer.

The adhesive compositions useful herein may include, in addition to one or more polymers as described above, conventional additives such as tackifiers (wood rosin, polyesters, etc.), antioxidants, fibrous or nonfibrous fillers, and colorants. It is also possible to include additional adhesives, providing that the overall temperature sensitivity profile is not significantly affected.

Coating of the body member with the adhesive composition may be done in any number of ways, e.g., by spray deposition, painting, dipping, gravure printing, rolling, or transfer from a release sheet, in a manner similar to that involved in transfer printing.

### EXAMPLES

The invention is illustrated in the following examples, in which parts, percentages and proportions are by weight and in which the following abbreviations and test methods are employed. AA is acrylic acid, MA is methyl acrylate, EA is ethyl acrylate, C6A is hexyl acrylate, C16A is hexadecyl acrylate, PGMA is polyethylene glycol monomethyl ether methacrylate, the polyethylene glycol monomethyl ether having a degree of polymerization of 8, PTHF is polytetrahydrofuran having a molecular weight of 2900 (available from Scientific Polymer Products, Ontario, New York), XAMA is trimethylolpropane-tris-[B-(N-aziridinyl) propionate (available from Virginia Chemicals, Portsmouth, VA nder the trade name XAMA 2), AIBN is azobisisobutyronitrile, and Tuftane is a 0.3 mm (0.012 inch) thick polyurethane film (available from Lord Corporation, Erie, PA, under the trade name Tuftane 410).

IMTI peel strength was measured as follows. A solution (50% solids) of the adhesive formulation was prepared in tetrahydrofuran, cast onto a flexible PVC film, and air-dried at 70°C for 30 minutes. A 2.54 cm (1 inch) wide strip was covered with a 2.54 cm (1 inch) uncoated strip of the PVC film. The assembly was lightly pressed together at a temperature of 37°C. Average peel strength was measured using an Instron materials testing instrument equipped with a variable temperature chamber and at a speed of 25.4 cm/min (10 inch/min).

ISPI peel strength was measured as follows. An Instrumentors Inc. SP-102B Slip/Peel Tester was used according to the test procedure designated as PCTC-1 (revision 8/85) by the Pressure Sensitive Tape Council and was calibrated and operated according to the instruction manual accompanying the instrument. The coating was cast from a heptane/methyl ethyl ketone solution (90:10) containing between 15% and 45% of the adhesive, dried in an oven for 18 to 24 hours at 50°C and cooled for 0.50 to 0.75 hours at room temperature. Specimens in the form of strips 12.5 x 125 mm (0.5 x 5.0 inch) were cut lengthwise in the direction of extrusion of the backing substrate. The coating thickness was measured at 3 or more points and averaged to obtain a thickness for each strip. The average of the specimen thickness values, to the nearest 0.025 mm (0.001 inch), was the reported coating thickness. The peel test was conducted as soon as possible after measuring the coating thickness. The testing substrate was Tuftane, secured to the temperature-controlled platen by means of double-sided carpet tape. Each specimen remained on the testing surface for 5 minutes before testing at the prescribed temperature of testing. Ten specimens were tested at each temperature. Each specimen was rolled with a 2 kg (4.5 pound) rubber roller immediately after contact with testing surface. The roller traveled over the surface of the specimen at a rate of 305 mm/min (12 inches/min) in both directions. The average peel force and terminal surface temperature were recorded.

STA tack was measured using a surface texture analyzer (a Voland-Stevens-LFRA texture analyzer). The probe was allowed to contact the adhesive for 10 seconds and was then withdrawn at 0.2 mm/sec. STA tack values reported herein are the average maximum reading.

PKI tack was measured by ASTM D2979. This test is carried out at room temperature using a Polyken instrument, and measures in g.cm/sec the force required to remove the end of a stainless steel rod, 5.0 mm in diameter, from the surface of an adhesive-coated assembly. Prior to the removal, the rod approaches the adhesive at a speed of 10 mm per second and makes contact with the adhesive for 0.5 second.

Tack temperature was measured as follows. A 2.5 x 2.5 mm (1 inch x 1 inch) test sample was bonded face up to a metal plate with double-sided adhesive tape and the metal plate was placed in a temperature-controlled oven and allowed to equilibrate at the selected temperature for 10 minutes. Tack was tested by lightly pressing a 1 cm diameter plastic rod onto the surface of the adhesive for 1 second and then removing. After testing at the lowest temperature, the oven temperature was increased by 2°C and test repeated. The tack temperature is defined as the lowest temperature at which a noticeable tack was observed.

### Example 1

C16A (10 g), EA (2 g), AIBN (0.06 g) and toluene (15 ml) were heated at 60°C under nitrogen for 12 hours. The resulting mass was extracted with ethanol and dried in vacuo to yield a rubbery mass having a Tₒ of 34°C, a T_{c} of 26°C and a ΔH_{f} of 64 Jg. A sample of this material was heated to 70°C and pressed into a 0.025 mm (0.002 inch) thick film. A sample of the film was placed onto the adhesive side of a commercial plastic-backed PSA tape and stored at 25°C. The resulting tape exhibited no tack or adhesion to paper at room temperature. When the tape was placed on the wrist of a human subject, however, it became tacky almost instantly and exhibited good adhesion. When removed from the skin and kept at room temperature, the tape quickly lost its tack and adhesive properties.

### Example 2

A mixture of a copolymer of C16A, AA and EA (90:5:5)(1 g), toluene (1 ml) and XAMA (0.004 g) was allowed to stand for two days at 80°C. More toluene was added to make the resulting viscous solution spreadable. The resulting mixture was spread onto clear PVC film, dried at 80°C for 1 hour, and allowed to cool. The resulting layer exhibited no tack at room temperature, but displayed excellent adhesion to skin at body temperature, and was easily removed with a cool, damp paper towel.

### Example 3

A mixture of C16A (16 g), isodecyl acrylate (3 g), AA (1 g), AIBN (0.1 g) and toluene (30 ml) was purged with nitrogen and reacted for 11 hours at 70°C. The reaction mixture was precipitated into methanol and the resulting polymer dried. A 50% solids solution of the polymer in tetrahydrofuran was prepared. One portion of the solution was coated onto a PVC backing and dried at 50°C to give a coating about 0.025 mm thick. Other portions of the solution were treated with XAMA (0.35% w/w or 0.75% w/w) before being coated onto a PVC backing and dried in the same way, resulting in crosslinked adhesives.

The STA Tacks and IMTI Peel Strengths of these coatings were measured at 20°C and 39°C and are shown in Table I.

**Table I**

| | STA TACK (g/cm2) | | IMTI PEEL STRENGTH (g/cm) | |
|---|---|---|---|---|
| % w/w XAMA | 20°C | 39°C | 20°C | 39°C |
| | | | | |
| 0 | 0 | >100 | <4.5 | 7 |
| 0.75 | 0 | 12 | <4.5 | -- |
| 0.35 | 0 | 15 | <4.5 | 21 |

### Example 3a

C16A, C6A and AA, in the proportions shown in Table II, were copolymerized, using AIBN as a catalyst, to prepare copolymers having the molecular weights shown in Table II.

**Table II**

| Copolymer Sample | Monomer Content (%) | | | Mol. Weight Mw (K=1000) |
|---|---|---|---|---|
| | C16A | C6A | AA | |
| | | | | |
| 1 | 83.5 | 12.5 | 4 | 914 K |
| 2 | 83.5 | 12.5 | 4 | 862 K |
| 3 | 83.5 | 12.5 | 4 | 739 K |
| 4 | 83.5 | 12.5 | 4 | |
| 5 | 84 | 13 | 3 | 401 K |
| 6 | 25 | 72 | 3 | 999 K |

None of samples 1-5 began melting before 27.5°C and all had completed melting by 34.5°C. ΔH_{f} values of samples 1-5 were from 44 to 52 J/g. Sample 6 had no detectable first order transition above 5°C.

### Example 3b

A blend was prepared of lauric acid and the copolymers 1-5 prepared in Example 3a, in the amounts shown in Table III below.

**Table III**

| | |
|---|---|
| Sample 1 | 12.73 |
| Sample 2 | 13.26 |
| Sample 3 | 21.93 |
| Sample 4 | 9.02 |
| Sample 5 | 39.62 |
| Lauric Acid | 3.39 |

### Example 3c

A dodecene polyolefin was prepared as follows. Dry heptane (100 ml) was added to a dried, 2 liter reaction flask under a blanket of dry nitrogen. Catalyst was prepared by adding 0.7 ml of TiCl₄ and 3 ml of A1(C₂H₅)₃ to the heptane. The system was stirred for one hour at room temperature (a bath was used to ensure a constant temperature) and purged with dry nitrogen to remove all traces of oxygen. Next, 240 ml (180 g) of dodecene was added to the flask in as anhydrous a manner as practical. The polymerization reaction was run for 5 hours. At the end of this time, the reaction was quenched with ethanol. The polymer precipitated and the solution was decanted. The precipitate was washed with fresh alcohol and then dissolved in toluene at temperatures not exceeding 90°C. That solution was again precipitated by adding an excess of ethanol. That material was filtered and dried in a vacuum oven at room temperature. The final dried product weighed 89 grams. The viscosity of the polymer was measured in cyclohexane. The values of the inherent and reduced specific viscosities were extrapolated to zero concentration and found to be 4.22 and 3.79 respectively. DSC analysis revealed that the polymer sample had a broad melting range beginning at about 31°C and ending at about 52°C, and had a ΔH_{f} of 28 J/g.

### Example 3d

The following experiment demonstrates how temperature affects the tack of various adhesives. A commercial surgical incise drape (sold by 3M Corporation under the trade name Steri Draft 1040) was used as a control; the adhesive coating on this drape was 0.025 mm (0.001 inch) thick. The SCC polymer blend of Example 3b was cast on a duPont Hytrel™ backing using a 90:10 mixture of heptane:methylethyl ketone as a solvent, and dried, resulting in an adhesive coating 0.045 mm (0.00175 inch) thick. The polydodecene prepared in Example 3c was cast on Mylar polyester film from a solution of heptane and dried, resulting in an adhesive coating 0.0635 mm (0.00250 inch) thick.

The PKI Tack values of the Control and of the two other adhesive coatings were measured at various temperatures in the range 23° to 45°C as the temperature was raised (i.e. the tack was measured at a temperature which had not previously been exceeded). The measured tack values of the Example 3b adhesive were reduced by a factor of 1/1.75, and the measured tack values of the Example 3c adhesives were reduced by a factor of 1/2.50, thus normalizing them to take into account the different adhesive thicknesses and making the tack values comparable. Table IV shows the normalized PXI tack values of the samples.

**Table IV**

| Temperature (°C) | Control | Example 3b | Example 3c |
|---|---|---|---|
| | | | |
| 23 | | | 14.66 |
| 25 | 90.77 | | 14.66 |
| 26 | 123.92 | 21 | 14.66 |
| 27 | 72.29 | 21 | 14.66 |
| 28 | 52.62 | 26.63 | 15.25 |
| 28 | 71.16 | 211.26 | 29.07 |
| 29 | 71.16 | 211.26 | 29.07 |
| 30 | 97.99 | 273.43 | 38.77 |
| 31 | 75.31 | 297.71 | |
| 32 | 57.94 | | 59.05 |
| 35 | | 470.82 | 57.00 |
| 40 | | | 84.02 |
| 45 | | | 390.2 |

Table IV shows that the Control was always tacky or slightly tacky and was not significantly affected by the change in temperature. The multitactic polyolefin of Example 3c was nontacky below 28°C, became slightly tacky when the temperature was raised above 28°C and then became progressively more and more tacky as the temperature was raised to 45°C. In contrast, the blend in Example 3b was nontacky below 28°C and then became tacky above 28°C.

### Example 3e

This Example shows how the PKI tacks of the adhesives of Examples 3b and 3c vary with time after the adhesive has been heated to 70°C and then cooled to 23°C. Fresh samples, prepared as described in Examples 3d, were used for this study. The samples were conditioned at 70°C for 2 hours, and then cooled to 23°C. Table V below lists the normalized tack values of the samples, all measured at 23°C.

**Table V**

| Time (hours) | Example 3b | Example 3c |
|---|---|---|
| 0.03 | 28.14 | >400.00 |
| 0.08 | 20.94 | >400.00 |
| 0.17 | 20.94 | >400.00 |
| 0.50 | 20.94 | >400.00 |
| 1.00 | 20.94 | >400.00 |
| 2.00 | 20.94 | >400.00 |
| 5.00 | 20.94 | >400.00 |
| 8.00 | 20.94 | 172.84 |
| 24.00 | 20.94 | 94.78 |
| 48.00 | 20.94 | 90.40 |
| 72.00 | 20.94 | 75.85 |
| 120.00 | 20.94 | 55.78 |
| 126.00 | 20.94 | 53.07 |
| 127.00 | 20.94 | 36.58 |
| 144.00 | 20.94 | 32.30 |
| 152.00 | 20.94 | 39.65 |
| 169.00 | 20.94 | 33.98 |
| 177.00 | 20.94 | 30.48 |
| 192.00 | 20.94 | 40.12 |
| 199.00 | 20.94 | 22.55 |
| 200.00 | 20.94 | 27.60 |
| 216.00 | 20.94 | 32.90 |
| 240.00 | 20.94 | 21.16 |
| 247.00 | 20.94 | 15.60 |
| 248.00 | 20.94 | 14.66 |

Table V shows that a high degree of tack is retained by sample 3c for several days, whereas the 3b sample regains its non-tacky state within 5 minutes.

### Example 3f

Table VI shows how the normalized ISPI peal strengths of the adhesive of Example 3b and of the commercially available control, directly after application, vary with the temperature at which they are affixed to the polyurethane substrate, and how the normalized ISPI peal strengths, measured after the substrate and the adhesive have been cooled to 20°C for 5 minutes, vary with the temperature of affixation to the substrate.

**Table VI**

| Affix Temperature (°C) | Sample 3b at Affix Temp | Sample 3b at 20°C | 3M Control at Affix Temp | 3M Control at 20°C |
|---|---|---|---|---|
| | | | | |
| 38.0 | | 30.00 | | 111.67 |
| 37.1 | 79.28 | | | |
| 37.0 | | | 87.36 | |
| 36.0 | | 28.73 | | 107.74 |
| 34.0 | 67.68 | | | |
| 33.8 | | | 89.55 | |
| 33.0 | | 11.34 | | 107.85 |
| 32.0 | 67.69 | | 90.69 | |
| 31.0 | | 12.85 | | 99.17 |
| 30.2 | | | 89.88 | |
| 30.1 | 52.95 | | | |
| 28.0 | 28.45 | | | |
| 27.9 | | | 100.16 | |
| 26.0 | 0.00 | | | |
| 25.7 | | | 101.81 | |
| 22.7 | 0.00 | | 110.98 | |

Table VI shows that the peel strength of the control does not vary much with the temperature of affixation and is not decreased by cooling. By contrast, the peel strengths of 3b samples demonstrate a substantial change at affixation temperatures of about 28°C, and undergo a significant reduction when cooled to 20°C after being affixed at any temperature above 28°C.

### Example 4

Pentadecyl acrylate (19 g), AA (1 g), heptane (20 ml), ethyl acetate (10 ml) and AIBN (0.07 g) were combined. The solution was degassed and mixed at 70°C for 17 hours. The resulting polymer was isolated and dried in vacuo.

The polymer (1 g) and XAMA (0.003 g) were dissolved in 2 ml of 1:1 heptane:ethylacetate, and the solution coated onto a 0.005 cm thick polyurethane backing (2103 AE, Dow Chemical, Midland, Michigan) and dried at 80°C for 15 minutes. The resulting tape was nontacky at room temperature but quickly adhered upon application to skin. During a 36-hour test on a human subject no disbondment of adhesive or film from skin was observed.

### Example 5

A copolymer of C16A, EA and AA (76.5:20:2.5) (2 g), and XAMA (0.0043 g) were dissolved in 4 ml of 1:1 ethylacrylate:heptane. The resulting solution was coated onto a polyurethane film and dried at 110°C for 15 minutes. The resulting film was not tacky at 25°C but bonded readily to a human subject. Samples showed no loss of adhesion when subjected to washing. During a 36-hour test, no delamination of adhesive was observed.

### Example 6

A solution of the polymer of Example 3 was coated onto a polyurethane backing and dried at 100°C. The same procedure was followed with a solution containing 0.3% XAMA, thus preparing a crosslinked adhesive.

### Example 7

A copolymer of C16A, MA and AA (85:10:5) was prepared as in Example 3. The polymer (1 g), XAMA (0.024 g) was combined with heptane (1 ml), ethyl acetate (1 ml) and coated onto a polyurethane backing and cured as in Example 6 above. Six 2.54 cm x 2.54 cm test samples were applied to a human test subject for 26 hours. Three were removed at ambient temperature and three were chilled for 20 seconds with a cold can of soda prior to removal. All of the samples removed at ambient temperature resulted in visible amounts of skin being removed from the test subject. Two of the cooled samples showed no visible skin removal, while the third showed a very small amount of skin removal.

### Example 8

C16A (43 g), EA (6 g), AA (1 g) and AIBN (0.5 g) were dissolved in ethyl acetate (150 g). The solution was purged with nitrogen and was heated at 45°C for 24 hours and then at 60°C for 2 hours. The viscous reaction product was precipitated into chilled ethanol. After drying in a vacuum oven, the material was dissolved into heptane (30% solids).

An ionic crosslinking agent, zinc stearate (1% based on polymer) was added to one-half the solution. The other half of the solution was used to cast film on Tuftane. The resultant dried film was 0.019 (0.0075 inch) thick. Strips, 12.7 x 38 mm (0.5 x 1.5 inch) were cut from the sheet and mounted on the skin surface. They immediately were transformed from a non-tacky to an aggressively tacky strip. The strips were worn for 5 hours before they were removed at a rate of 1 cm/sec. A Chatillon DFG-2 Digital Gram Gauge was used to monitor the force needed for removal under normal conditions and after the sample had been cooled with a cool damp cloth for several minutes. The uncooled removal required a peak force of about 0.273 Kg, while the chilled removal required a peak force of about 0.132 Kg. The uncooled removal used 2.25 times more energy than the cooled removal.

The solution containing zinc stearate was used in the same way except that half the samples were chilled with an ice cold can of soda. The average force required to remove the uncooled samples was 0.156 Kg, while the force to remove the chilled samples was only 0.036 Kg.

### Example 9

The crosslinked adhesive-coated urethane of Example 5 was applied to Tuftane and warmed to 35°C. The material bonded well at this temperature, but exhibited no adhesion when cooled to 20°C.

### Example 10

A copolymer of octadecylmethacrylate and AA (97.5:2.5) (1 g) and XAMA (0.0028 g) were dissolved in ethyl acetate (1 ml) and coated onto a polyurethane backing as in Example 6. Four 25 x 25 mm (1 x 1 inch) samples were applied to a human test subject. After 24 hours, two of the samples were removed at room temperature, and two were first cooled with a cold can of soda and then removed. The two samples removed at ambient temperature showed visible skin removal, while the samples which had been cooled showed no visible skin removal.

### Example 11

C16A (4.25 g), tetradecyl acrylate (4.24 g), PGMA (1.02 g), AA (0.5 g), AIBN (0.033 g) and toluene (20 ml) were maintained at 60°C for 14 hours. A sample of the resultant polymer was combined with 0.25% XAMA and coated onto a polyurethane backing as in Example 6.

### Example 12

PTHF (31 g), hexamethylene diisocyanate (1.85 g), dibutyltin dilaurate (1 drop) and toluene (200 ml) were combined and mixed for 24 hours. Ethanol (5 ml) was then added with stirring. The resultant solution was coated onto a glass slide, dried at 100°C for 1 hour and then allowed to cool overnight. The resulting film was nontacky at 10°C but tacky at skin temperature yet showed no tendency to flow.

### Example 13

PTHF (1 g) and 3.0 grams of the polymer solution of Example 12 were combined and coated onto a glass microscope slide, dried at 100°C for 12 hours and allowed to cool.

### Example 14

C16A (8.5 g), PGMA (1 g), AA (0.5 g), AIBN (0.0667 g) were dissolved in toluene (20 ml). The solution was purged with nitrogen and heated at 60°C for 14 hours. The product was precipitated into ethanol, filtered and dried under vacuum.

Test samples were prepared by solution casting. The solution contained 2 grams of the above polymer and enough ethyl acetate to bring the total weight up to 6 grams. To portions of this solution was added 0, 1, 2 or 3% of XAMA. Each solution was cast on polyvinyl chloride film using a 87 mm wide blade set at 0.076 mm (0.003 inch). The films were air dried and then heated for 1 hour at 58°C. The widths of the films were 81, 76, 73 and 68 mm, respectively, indicating that crosslinking had occurred to various degrees.

Each of the samples became tacky above a temperature of 36°C, and the tackiness decreased as the amount of XAMA 2 was increased.

### Example 15

A polymer solution was prepared as in Example 13 with C16A, EA and AA in an 80:15:5 ratio. Testing was carried out as described in Example 14.

### Example 16

The tack temperatures of the compositions of Examples 3, 4, 5, 7, 11 and 15 are set forth in Table VII.

**Table VII**

| Composition | % XAMA (% ww) | Tack Temperature |
|---|---|---|
| Example 3 | 0 | 31 |
| Example 3 | 0.35 | 29 |
| Example 4 | 0.3 | 31 |
| Example 5 | 0.22 | 29 |
| Example 7 | 0 | 33 |
| Example 7 | 0.30 | 33 |
| Example 11 | 0.25 | 33 |
| Example 15 | 0.25 | 29 |

### Example 17

A copolymer of C16A, C6A and AA (84:13:3) (26 parts) was dissolved in heptane (66.6 parts) and methyl ethyl ketone (74 parts). The solution was cast as a film on a release liner backing of siliconized polypropylene paper. After air drying, the film thickness was about 0.25 mm (0.01 inch)thick. The film was heated in a convection oven for 18 hours at 50°C. The final dried adhesive film was about 0.06 mm (0.0025 inch) thick and exhibited a melting temperature of 29°C.

The film assembly produced above was placed with the adhesive face down on Whatman paper stock #4. The assembly was heated to about 35°C and rolled with a standard 2 kg (4.5 lb) roller. The adhesive film formed a strong bond with the Whatman paper, as evidenced by the fact that the release liner could be separated from the adhesive film, leaving the adhesive film attached to the paper.

Designs were drawn on the adhesive-backed paper while it was maintained at room temperature (approximately 25°C). The release liner was then removed. The adhesive coating was smooth and tack-free. Small shapes corresponding to the designs were cut from the coated Whatman paper, and placed on a sheet of white bond paper, adhesive side down.

The cut-out shapes could be repositioned easily because the adhesive was not at all tacky. When the arrangement of the shapes was satisfactory, they were covered by a thin piece of cardboard, and the assembly was placed between two steel plates and then in an oven at 35°C for 5 minutes. The steel plate acted as a gentle press. The assembly was then disassembled and the white bond paper examined at about 24°C. The cut-out shapes were permanently secured to the bond paper. Even when the paper was placed in the refrigerator, the shapes remained in place and were stable.

### Example 18

40 parts of a first polymer and 60 parts of a second polymer were mixed. The first polymer was a copolymer of C16A, C6A and AA (84:13:3) and had an M_{w} (in tetrahydrofuran) of 1.9 x 10⁶. The second polymer was a copolymer of C16A, isodecylacrylate and AA (74:20:6) and had an M_{w} (in toluene) of 50,000. 25 parts of this polymer mixture were dissolved in 75 parts of a 90:10 mixture of hexane and methylethylketone, and the solution was cast as a film on a release liner backing of siliconized polypropylene paper, using an appropriate wire-wound coating rod. After air drying, the film assembly was heated in a convection oven for 18 hours at 50°C. The final dried adhesive film was 0.0032 mm (0.00125 inch) thick and exhibited a melting temperature of 21°C. The coating process and drying process were repeated to produce a final dried adhesive film thickness of 0.064 mm (0.0025 inch).

The assembly produced above was placed with the adhesive face down on Whatman paper stock #4. The assembly was heated to about 40°C and rolled with a standard 2 kg (4.5 lb) roller to cause the adhesive polymer to flow into the interstices of the cellulosic fibers of the paper. The adhesive film had formed a strong bond with, and had indeed transferred to, the paper during this process, as evidenced by the fact that the release liner could be separated from the adhesive film, leaving the adhesive film attached to the paper.

The assembly was cut into strips about 9.5 x 51 mm (0.375 x 2 inch). The release liner was removed from the strips, which were then applied to selected substrates at about 24°C. The substrates were test tubes, synthetic leather, plastic and metal pens, painted wood pencils and a small varnished article of wood.

The strips bonded well to each of the substrates. Attempts to remove them at 24°C resulted in tearing of the paper. This result demonstrates the value of such strips as tamper-proof labels.

After the label-bonded substrates had been maintained at 5°C for 15 minutes, the labels could be easily removed without tearing the paper, and without leaving any adhesive residue on the substrate.

## Claims

1. A temperature-sensitive adhesive assembly which comprises
(1) a body member; and
(2) secured to the body member, a layer of a polymeric composition which
(a) comprises 40 to 100% by weight of at least one crystalline polymer which (i) begins to melt at a temperature Tₒ which is 5 to 50°C, and (ii) completes melting at a temperature Tₘ which is at most (Tₒ+15)°C,
(b) is substantially non-tacky at temperatures below Tₒ, and
(c) becomes tacky when heated from a temperature below Tₒ to a temperature above Tₘ.

2. An assembly according to claim 1 wherein the polymer has a Tₒ of at least 25°C, preferably 25 to 30°C, and Tₘ is at most (Tₒ+10)°C, preferably at most (Tₒ+5)°C.

3. An assembly according to claim 1 or 2 wherein the polymer has a crystallization point of 10 to 28°C.

4. An assembly according to claim 2 or 3 wherein the polymer is a side chain crystallizable polymer.

5. An assembly according to claim 4 wherein the side chain crystallizable polymer contains units derived from at least two monomers.

6. An assembly according to claim 4 or 5 wherein the crystalline polymer is a side-chain crystallizable polymer which has a heat of fusion of at least 20 Joules/g and contains at least 50% by weight of repeating units of the formula 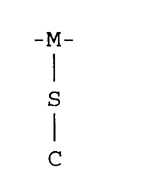 wherein
M is an atom forming part of the backbone of the polymer,
S is a spacer unit, and
C is a crystallizable group.

7. An assembly according to claim 6 wherein said repeating units are derived from at least one n-alkyl acrylate or methacrylate in which the alkyl group contains 14 to 22 carbon atoms.

8. An assembly according to claim 6 or 7 wherein the polymer includes repeating units derived from a monomer selected from acrylic acid, hydoxyethylacrylate, methacrylic acid, styrene, and substituted styrenes.

9. An assembly according to any one of claims 4 to 8 wherein the polymer contains 1 to 10% by weight of units derived from acrylic acid.

10. An assembly according to any one of claims 4 to 9 wherein the polymer contains polyoxyethylene, polyoxypropylene or polyoxybutylene units.

11. An assembly according to claim 2 or 3 wherein the polymer is a polyolefin of singular tacticity.

12. An assembly according to any one of the preceding claims wherein the crystalline polymer is a block copolymer containing a crystallizable portion and a second portion which exhibits a glass transition point or melting point higher than the crystallizable portion.

13. An assembly according to any one of claims 1 to 10 wherein the crystalline polymer is crosslinked and has a heat of fusion of at least 20 J/g, preferably at least 30 J/g.

14. An assembly according to any one of the preceding claims wherein the polymeric composition contains a tackifier, a fibrous filler, a non-fibrous filler, or a colorant.

15. An assembly according to any one of the preceding claims wherein the layer of polymeric composition is slightly tacky at room temperature and the assembly includes a release liner which protects the layer.

16. An assembly according to any of claims 2 to 11 wherein the body member is a woven fabric, a non-woven fabric, paper, a synthetic film, a surgical dressing, an adhesive bandage with a gauze pad in the center, a transdermal drug delivery patch, an electrode, a cast or a splint.

17. An assembly according to any of claims 1 to 15 wherein the body member is a surgical dressing, an adhesive bandage with a gauze pad in the center, a transdermal drug delivery patch, an electrode, a cast, or a splint.

18. An assembly according to any of claims 1 to 15 wherein the body member is a flexible article.

19. An assembly according to any of claims 1 to 15 wherein the body member is a non-woven fabric or paper.

20. An assembly according to any of claims 1 to 15 wherein the body member is a synthetic film.

21. A method of securing a body member to a substrate which comprises pressing against the substrate the layer (2) of a temperature-sensitive adhesive assembly as claimed in any one of the preceding claims while said layer is tacky.

22. A method of removing a body member from a substrate, the body member being part of a temperature-sensitive assembly as claimed in any one of claims 1 to 20 which is adhered to the substrate through the layer (2) of the assembly, which method comprises
(A) cooling the assembly to a temperature at which the layer (2) is substantially non-tacky; and
(B) removing the body member from the substrate.

23. The use of a temperature-sensitive adhesive assembly as claimed in any one of claims 1 to 20 to secure the body member to human skin.

## Patentansprüche

1. Temperaturempfindliche klebende Anordnung, die
(1) einen Grundkörper und
(2) eine an dem Grundkörper befestigte Schicht einer polymeren Zusammensetzung umfaßt, die
(a) 40 bis 100 Gew.-% wenigstens eines kristallinen Polymers enthält, das (i) bei einer Temperatur Tₒ zu schmelzen beginnt, die 5°C bis 50°C beträgt und (ii) bei dem der Schmelzvorgang bei einer Temperatur Tₘ, die höchstens (Tₒ + 15)°C beträgt, abgeschlossen ist,
(b) bei Temperaturen unterhalb Tₒ im wesentlichen nicht klebrig ist und
(c) klebrig wird, wenn sie von einer Temperatur unterhalb Tₒ auf eine Temperatur über Tₘ erwärmt wird.

2. Anordnung nach Anspruch 1, bei der Tₒ des Polymers wenigstens 25°C, vorzugsweise 25°C bis 30°C, und Tₘ höchstens (Tₒ + 10)°C, vorzugsweise höchstens (Tₒ+5)°C, ist.

3. Anordnung nach Anspruch 1 oder 2, in der das Polymer einen Kristallisationspunkt von 10°C bis 28°C aufweist.

4. Anordnung nach Anspruch 2 oder 3, in der das Polymer ein Seitenkettenkristallisierbares Polymer ist.

5. Anordnung nach Anspruch 4, in der das Seitenketten-kristallisierbare Polymer Einheiten enthält, die von wenigstens zwei Monomeren abgeleitet sind.

6. Anordnung nach Anspruch 4 oder 5, in der das kristalline Polymer ein Seitenkettenkristallisierbares Polymer ist, das eine Schmelzwärme von wenigstens 20 Joules/g aufweist und wenigstens 50 Gew.-% der sich wiederholenden Einheiten der Formel 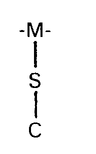 enthält, in der
M ein atombildender Teil der Hauptkette des Polymers,
S eine zwischengeschaltete Einheit und
C eine kristallisierbare Gruppe ist.

7. Anordnung nach Anspruch 6, in der die sich wiederholenden Einheiten wenigstens von einem n-Alkylacrylat oder Methacrylat abgeleitet sind, in denen die Alkylgruppe 14 bis 22 Kohlenstoffatome enthält.

8. Anordnung nach Anspruch 6 oder 7, in der das Polymer sich wiederholende Einheiten enthält, die von einem Monomer abgeleitet sind, das ausgewählt wird aus Acrylsäure, Hydroxyethylacrylat, Methacrylsäure, Styrol und substituierten Styrolen.

9. Anordnung nach einem der Ansprüche 4 bis 8, in der das Polymer 1 bis 10 Gew.-% Einheiten enthält, die von Acrylsäure abgeleitet sind.

10. Anordnung nach einem der Ansprüche 4 bis 9, in der das Polymer Polyoxyethylen-, Polyoxypropylen- oder Polyoxybutyleneinheiten enthält.

11. Anordnung nach Anspruch 2 oder 3, in der das Polymer ein Polyolefin mit singulärer Taktizität ist.

12. Anordnung nach einem der vorhergehenden Ansprüche, in der das kristalline Polymer ein Blockpolymer ist, das einen kristallisierbaren Anteil und einen zweiten Anteil enthält, der einen Glasumwandlungspunkt oder einen Schmelzpunkt höher als der des kristallisierbaren Anteils besitzt.

13. Anordnung nach einem der Ansprüche 1 bis 10, in der das kristalline Polymer quervernetzt ist und eine Schmelzwärme von wenigstens 20 J/g, vorzugsweise 30 J/g, aufweist.

14. Anordnung nach einem der vorhergehenden Ansprüche, in der die polymere Zusammensetzung ein Mittel zum Klebrigmachen, einen fibrösen Füller, einen nichtfibrösen Füller oder einen farbgebenden Stoff enthält.

15. Anordnung nach einem der vorhergehenden Ansprüche, in der die Schicht der polymeren Zusammensetzung bei Raumtemperatur leicht klebrig ist und die Anordnung einen abziehbaren Liner umfaßt, der die Schicht schützt.

16. Anordnung nach einem der Ansprüche 2 bis 11, in der der Grundkörper ein gewebter Stoff, ein nichtgewebter Stoff, Papier, ein synthetischer Film, ein chirurgischer Verband, eine Klebebandage mit einem Gazebausch in der Mitte, ein transdermales Arzneimittelfreisetzungspflaster, eine Elektrode, ein Gipsverband oder eine Schiene ist.

17. Anordnung nach einem der Ansprüche 1 bis 15, in der der Grundkörper ein chirurgischer Verband, eine Klebebandage mit einem Gazebausch in der Mitte, ein transdermales Arzneimittelfreisetzungspflaster, eine Elektrode, ein Gipsverband oder eine Schiene ist.

18. Anordnung nach einem der Ansprüche 1 bis 15, in der der Grundkörper ein flexibler Artikel ist.

19. Anordnung nach einem der Ansprüche 1 bis 15, in der der Grundkörper ein nichtgewebtes Gewebe oder Papier ist.

20. Anordnung nach einem der Ansprüche 1 bis 15, in der der Grundkörper ein synthetischer Film ist.

21. Verfahren zur Befestigung eines Grundkörpers an einem Substrat, wobei das Verfahren das Pressen der Schicht (2) einer temperaturempfindlichen klebenden Anordnung nach einem der vorhergehenden Ansprüche gegen das Substrat umfaßt, wenn die Schicht klebrig ist.

22. Verfahren zur Entfernung eines Grundkörpers von einem Substrat, wobei der Grundkörper Teil einer temperaturempfindlichen Anordnung nach einem der Ansprüche 1 bis 20 ist, die auf dem Substrat durch die Schicht (2) der Anordnung befestigt ist, wobei das Verfahren die folgenden Schritte umfaßt:
(A) Abkühlen der Anordnung auf eine Temperatur, bei der die Schicht (2) im wesentlichen nicht klebrig ist, und
(B) Entfernen des Grundkörpers von dem Substrat,

23. Verwendung einer temperaturempfindlichen klebrigen Anordnung nach einem der Ansprüche 1 bis 20 zur Befestigung des Grundkörpers auf menschlicher Haut.

## Revendications

1. Ensemble adhésif sensible à la température, comprenant :
(1) un corps ; et
(2) une couche d'une composition à base de polymère, fixée au corps, qui :
(a) comprend de 40 à 100 % en poids d'au moins un polymère cristallin qui (i) commence à fondre à une température Tₒ de 5 à 50 °C, et (ii) finit de fondre à une température Tₘ qui est au plus de (Tₒ+15) °C,
(b) est à peu près non adhésive à des températures inférieures à Tₒ, et
(c) devient adhésive lorsqu'elle est chauffée à partir d'une température inférieure à Tₒ jusqu'à une température supérieure à Tₘ.

2. Ensemble selon la revendication 1, dans lequel le polymère a une température de Tₒ d'au moins 25 °C, de préférence de 25 à 30 °C, et une température Tₘ d'au plus (Tₒ+10) °C, de préférence d'au plus (Tₒ+5) °C.

3. Ensemble selon la revendication 1 ou 2, dans lequel le polymère a un point de cristallisation de 10 à 28 °C.

4. Ensemble selon la revendication 2 ou 3, dans lequel le polymère est un polymère cristallisable à chaîne latérale.

5. Ensemble selon la revendication 4, dans lequel le polymère cristallisable à chaîne latérale, contient des motifs dérivés d'au moins deux monomères.

6. Ensemble selon la revendication 4 ou 5, dans lequel le polymère cristallin est un polymère cristallisable à chaîne latérale, ayant une chaleur de fusion d'au moins 20 J/g et contenant au moins 50 % en poids de motifs répétés de formule : 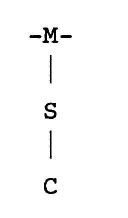 dans laquelle M représente un atome faisant partie du squelette du polymère,
S représente un motif d'espacement, et
C représente un groupe cristallisable.

7. Ensemble selon la revendication 6, dans lequel les motifs répétés sont dérivés d'au moins un acrylate ou un méthacrylate de n-alkyle dans lequel le groupe alkyle contient de 14 à 22 atomes de carbone.

8. Ensemble selon la revendication 6 ou 7, dans lequel le polymère comprend des motifs répétés dérivés de monomères choisis parmi l'acide acrylique, l'acrylate d'hydroxyéthyle, l'acide méthacrylique, le styrène et les styrènes substitués.

9. Ensemble selon l'une quelconque des revendications 4 à 8, dans lequel le polymère contient de 1 à 10 % en poids de motifs dérivés d'acide acrylique.

10. Ensemble selon l'une quelconque des revendications 4 à 9, dans lequel le polymère contient des motifs polyoxyéthylène, polyoxypropylène ou polyoxybutylène.

11. Ensemble selon la revendication 2 ou 3, dans lequel le polymère est une polyoléfine à tacticité simple.

12. Ensemble selon l'une quelconque des revendications précédentes, dans lequel le polymère cristallin est un copolymère séquencé contenant une partie cristallisable et une deuxième partie ayant un point de transition vitreuse ou un point de fusion supérieur à la partie cristallisable.

13. Ensemble selon l'une quelconque des revendications 1 à 10, dans lequel le polymère cristallin est réticulé, et a une chaleur de fusion d'au moins 20 J/g, de préférence d'au moins 30 J/g.

14. Ensemble selon l'une quelconque des revendications précédentes, dans lequel la composition à base de polymère, contient un agent adhésif, une charge fibreuse, une charge non fibreuse ou un colorant.

15. Ensemble selon l'une quelconque des revendications précédentes, dans lequel la couche de composition à base de polymère, est légèrement adhésive à la température ambiante, et l'ensemble comprend un revêtement anti-adhésif protégeant la couche.

16. Ensemble selon l'une quelconque des revendications 2 à 11, dans lequel le corps est un tissu tissé, un tissu non tissé, du papier, un film synthétique, un pansement chirurgical, un bandage adhésif comportant un tampon de gaz central, un timbre à libération de médicament percutanée, une électrode, un plâtre ou une attelle.

17. Ensemble selon l'une quelconque des revendications 1 à 15, dans lequel le corps est un pansement chirurgical, un bandage adhésif comportant un tampon de gaz central, un timbre à libération de médicament percutanée, une électrode, un plâtre ou une attelle.

18. Ensemble selon l'une quelconque des revendications 1 à 15, dans lequel le corps est un article souple.

19. Ensemble selon l'une quelconque des revendications 1 à 15, dans lequel le corps est un tissu non tissé ou un papier.

20. Ensemble selon l'une quelconque des revendications 1 à 15, dans lequel le corps est un film synthétique.

21. Procédé de fixation d'un corps sur un substrat, selon lequel on presse contre le substrat, la couche (2) d'un ensemble adhésif sensible à la température tel que revendiqué selon l'une quelconque des revendications précédentes, tandis que ladite couche est adhésive.

22. Procédé de séparation d'un corps à partir d'un substrat, le corps faisant partie d'un ensemble sensible à la température tel que revendiqué selon l'une quelconque des revendications 1 à 20, collé sur le substrat par l'intermédiaire de la couche (2) de l'ensemble, selon lequel :
(A) on refroidit l'ensemble jusqu'à une température à laquelle la couche (2) est à peu près non adhésive ; et
(B) on sépare le corps du substrat.

23. Utilisation d'un ensemble adhésif sensible à la température tel que revendiqué selon l'une quelconque des revendications 1 à 20, pour fixer le corps sur de la peau humaine.
